# EUROPÄISCHE PATENTSCHRIFT

(11) **EP 2 086 429 B1**
(45) Veröffentlichungstag und Bekanntmachung des Hinweises auf die Patenterteilung: **17.04.2013**
(21) Anmeldenummer: 07817651.8
(22) Anmeldetag: 15.10.2007
(51) Int. Cl.: A61B 17/32, A61F 9/007

(54) **MEDIZINISCHES HANDGERÄT**
HAND-HELD MEDICAL DEVICE
APPAREIL MÉDICAL MANUEL

(30) Priorität: 18.10.2006 DE 102006049702
(43) Veröffentlichungstag der Anmeldung: 12.08.2009
(73) Patentinhaber: Geuder AG, 69126 Heidelberg (DE)
(72) Erfinder: GEUDER, Volker, 69126 Heidelberg (DE)
(74) Vertreter: Ullrich & Naumann
(86) Internationale Anmeldenummer: PCT/DE2007/001809
(87) Internationale Veröffentlichungsnummer: WO 2008/046391

(56) Entgegenhaltungen:
- CH-A5- 671 872
- GB-A- 2 184 024
- US-A- 5 391 177
- US-A- 5 558 637
- US-A- 5 779 724
- US-B1- 6 884 240

## Beschreibung

Die Erfindung betrifft ein medizinisches Handgerät, insbesondere chirurgisches Schneidgerät zur Anwendung in der Ophthalmologie, mit einem als Griffstück dienenden Gehäuse und einem über ein Betätigungsorgan in dem Gehäuse verschiebbar angeordneten Werkzeughalter mit einem Werkzeug, insbesondere für ein Messer, wobei das Werkzeug durch Betätigung des Betätigungsorgans von einer Aufbewahrungsposition im Gehäuse in eine Arbeitsposition außerhalb des Gehäuses verschiebbar ist, wobei das Gehäuse zumindest im Bereich des in der Aufbewahrungsposition befindlichen Werkzeugs Öffnungen in der Gehäusewandung aufweist.

Medizinische Handgeräte sind in den unterschiedlichsten Ausprägungen aus der Praxis bekannt. Wesentlich ist dabei, dass das regelmäßig durch einen Werkzeughalter gehaltene Werkzeug von einer äußeren Arbeitsposition in eine im Gehäuse liegende Aufbewahrungsposition verbringbar ist.

Die aus der Praxis bekannten Handgeräte der gattungsbildenden Art sind in Bezug auf die Reinigung problematisch, da sich das verschmutzte Werkzeug in der Aufbewahrungsposition im Gehäuse befindet. Innerhalb des Gehäuses ist das Werkzeug jedoch nur bedingt zugänglich. Würde man das Werkzeug zum Zwecke der Reinigung in der Arbeitsposition außerhalb des Gehäuses belassen, bestünde die Gefahr der Beschädigung oder der Verletzung der handhabenden Person. Dies gilt insbesondere dann, wenn es sich bei dem Werkzeug um ein in den Werkzeughalter eingeklebtes Diamantmesser handelt. Dabei besteht nicht nur die Gefahr der Beschädigung, sondern auch die Gefahr des Verlustes der teuren Klinge.

Bislang wurden Handgeräte der hier in Rede stehenden Art in Ultraschallbädern gereinigt und anschließend sterilisiert. Mittlerweile ist erkannt worden, dass sich die Reinigung wesentlich einfacher in geeigneten Waschmaschinen vornehmen lässt. Jedoch eignen sich die gattungsbildenden Handgeräte kaum für eine solche Reinigung, zumal die voranstehend geschilderte Problematik besteht. Insbesondere beim Reinigen in einer Waschmaschine bestünde bei ungeschütztem Werkzeug eine erhöhte Gefahr der Beschädigung oder der Verlust der Klinge, so dass die an für sich einfache Reinigungsmethode in der Waschmaschine kaum oder nur bedingt anwendbar ist.

Zum Stand der Technik sei lediglich beispielhaft auf die EP 0 870 472 A1 verwiesen.

Aus der US 5,779,724 ist ein gattungsgemäßes medizinisches Handgerät mit einem als Griffstück dienenden Gehäuse bekannt. Über ein Betätigungsorgan ist ein Werkzeughalter in dem Gehäuse verschiebbar angeordnet. Das Werkzeug ist - ähnlich einem Kugelschreiber - durch Betätigung des Betätigungsorgans von einer Aufbewahrungsposition im Gehäuse in eine Arbeitsposition außerhalb des Gehäuses verschiebbar. Im Bereich des in der Aufbewahrungsposition befindlichen Werkzeugs sind Öffnungen in der Gehäusewandung vorgesehen.

Bei dem gattungsbildenden medizinischen Handgerät ist es während einer Operation möglich, von der Klinge zumindest grob Blut und andere Verunreinigungen abzuwaschen, während die Klinge im Gehäuse eingefahren ist. Da sich die Klinge dabei in dem eingefahrenen Zustand befindet, wird vermieden, dass der Operateur bzw. dessen Assistenten sich an der Klinge verletzen. Um das Handgerät nach einer Operation derart zu reinigen und zu sterilisieren, dass es nochmals bei einem weiteren Patienten eingesetzt werden kann, muss es jedoch komplett zerlegt und die Einzelteile gereinigt werden. Eine maschinelle Reinigung, die den hohen hygienischen Ansprüchen solcher medizinischer Instrumente genügt, ist nicht möglich.

Aus der GB 2 184 024 ist für sich gesehen ein chirurgisches Messer bekannt. Entsprechend den dortigen Ausführungen auf Seite 1, Zeilen 75 bis 79 ist dort kein in einem Gehäuse verschiebbar angeordneter Werkzeughalter vorgesehen.

Die US 5,391,177 A zeigt ein chirurgisches Schneidgerät zur Anwendung in der Ophthalmologie. Aus den Figuren 1 bis 3 und der dazugehörigen Beschreibung ist ersichtlich, dass in dem Gehäuse der Vorrichtung keine Öffnungen ausgebildet sind. Von einer maschinellen Reinigung des Handgeräts ist dort nicht die Rede.

Die DE 198 05 904 A1 zeigt ein chirurgisches Instrument mit einem Griffstück und einem Werkzeughalter. Das Griffstück ist gegenüber dem Werkzeughalter verschiebbar ausgeführt und über eine Arretiervorrichtung gegenüber dem Werkzeughalter arretierbar. Ein Betätigungsorgan; über das der Werkzeughalter in dem Gehäuse verschiebbar angeordnet ist, ist nicht offenbart. Außerdem ist dort nicht die Rede davon, dass das als Gehäuse dienende Griffstück über die gesamte Länge hinweg Öffnungen in der Gehäusewandung aufweist und dass die. Öffnungen allseitig im Gehäuse ausgebildet sind.

Die US 5,558,637 zeigt eine handgehaltene Vorrichtung zur Injektion eines Implantats in das Auge, wobei es sich dort um einen Wegwerfartikel handelt. Im Gehäuse ausgebildete Durchgänge bzw. Öffnungen sind äußerst klein ausgeführt und dienen ausschließlich zur Gas-Sterilisation vor der keimfreien Bereitstellung. Da es sich hier um einen Wegwerfartikel handelt, nämlich zum einmaligen Gebrauch, ist die Reinigung des Handgeräts nach Gebrauch nicht thematisiert und auch nicht möglich.

Der vorliegenden Erfindung liegt nun die Aufgabe zugrunde, ein medizinisches Handgerät der gattungsbildenden Art derart auszugestalten und weiterzubilden, dass es sich zur automatischen Reinigung in einer Waschmaschine eignet, ohne dabei das Werkzeug zu gefährden.

Die voranstehende Aufgabe ist erfindungsgemäß durch ein medizinisches Handgerät mit den Merkmalen des Patentanspruchs 1 gelöst. Danach ist das gattungsbildende Handgerät dadurch gekennzeichnet, dass die Öffnungen (5) allseitig, d.h. über den gesamten Umfang hinweg, im Gehäuse (1) ausgebildet sind.

Erfindungsgemäß ist erkannt worden, dass sich medizinische Handgeräte der hier in Rede stehenden Art zur Reinigung in Waschmaschinen nur dann eignen, wenn besondere konstruktive Maßnahmen realisiert sind. So weist das Gehäuse zumindest im Bereich des in der Aufbewahrungsposition befindlichen Werkzeugs Öffnungen in der Gehäusewandung auf, so dass der Sprüh- bzw. Reinigungsstrahl in der Waschmaschine das verschmutzte Werkzeug erreicht. Mit anderen Worten findet die Reinigung des Werkzeugs in der Aufbewahrungsposition des Werkzeugs statt, wobei die Öffnungen in der Gehäusewandung eine Wirkung des Reinigungsstrahls zulassen. Da es sich um räumlich begrenzte Öffnungen handelt, wird der Sprühstrahl, beispielsweise ausgehend von einem drehenden Sprüharm, regelmäßig gebrochen, so dass insoweit eine Art Entschärfung des Sprühstrahls stattfindet, ohne jedoch dessen Wirkung aufzuheben.

In erfindungsgemäßer Weise ist somit erkannt worden, dass sich das Werkzeug ohne weiteres in seiner Aufbewahrungsposition, d.h. innerhalb des Gehäuses des Handgeräts, reinigen lässt, wenn man die Gehäusewandung durchbricht bzw. Öffnungen vorsieht. Bei eingefahrenem Werkzeug, d.h. bei einem in der Aufbewahrungsposition befindlichen Werkzeug, ist das Werkzeug hinreichend gut geschützt und ist die Gefahr einer Verletzung ausgeschlossen. Somit erfüllt das Gehäuse nach wie vor seine schützende Funktion, lässt jedoch eine Reinigung des Werkzeugs von außerhalb zu. Demnach eignet sich das erfindungsgemäße Handgerät ganz besonders für die automatische Reinigung, insbesondere in einer eigens dafür vorgesehenen Spül- bzw. Waschmaschine.

Zur Gewährleistung einer kompletten Reinigung des Handgeräts, und zwar von außen und von innen, sieht die Erfindung vor, dass das Gehäuse über die gesamte Länge hinweg Öffnungen in der Gehäusewandung aufweist. So lässt sich nicht nur das Werkzeug sondern der gesamte Mechanismus innerhalb des Handgeräts reinigen und anschließend sterilisieren.

Im Konkreten weist das Gehäuse eine sich zum freien Ende hin vorzugsweise stetig verjüngende Gehäusespitze und einen vorzugsweise zylindrischen Gehäuseschaft auf. Insoweit ähnelt das Gehäuse dem Gehäuse eines Kugelschreibers. Die Öffnungen können sowohl in der Gehäusespitze als auch im Gehäuseschaft ausgebildet sein. Die Aufbewahrungsposition des Werkzeugs ist in einem solchen Falle in der Gehäusespitze lokalisiert, so dass den dortigen Öffnungen eine ganz besondere Bedeutung zukommt.

Die Öffnungen sind in weiter vorteilhafter Weise allseitig, d.h. über den gesamten Umfang des Gehäuses hinweg, im Gehäuse ausgebildet. Dabei ist es dankbar, dass die Öffnungen in axialer Richtung versetzt zueinander oder hintereinander fluchtend ausgebildet sind.

In Bezug auf die geometrische Ausgestaltung der Öffnungen sei an dieser Stelle angemerkt, dass diese beliebige Formen haben können. Insbesondere in Bezug auf eine sichere Handhabung, möglichst auch zur Vermeidung von Verletzungen im Bereich der Öffnungen, können diese rund bzw. mit gerundeten Randbereichen ausgebildet sein. Eine schlitzartige Ausgestaltung, vorzugsweise in ovaler Form, ist insoweit von Vorteil, dass sich dadurch relativ große Öffnungen generieren lassen, wobei es durch eine entsprechende Anzahl solcher Öffnungen möglich ist, dass das Gehäuse als eine Art Gehäusekäfig reduziert ist. Die käfigartige Ausbildung schützt das Innenleben, insbesondere das Werkzeug, und schütz auch den Benutzer vor Verletzungen. Durch den Gehäusekäfig ist das Werkzeug hinreichend gut in der Aufbewahrungsposition gesichert. Ein automatisches Reinigen ist möglich, zumal der Sprüh- bzw. Reinigungsstrahl - wenn auch gebrochen - in das Gehäuse bis hin zum Werkzeug gelangt und einen hinreichend guten Reinigungseffekt bewirken kann.

In weiter vorteilhafter Weise weist das Gehäuse bis hin zur Gehäusespitze einen zylindrischen Durchgang auf, in dem ein den Werkzeughalter tragender Kolben verschiebbar angeordnet ist. Der Kolben könnte durchgehend einheitlich ausgebildet sein. In vorteilhafter Weise verjüngt sich der Kolben zum Werkzeughalter hin, vorzugsweise über eine Art Abstufung. Im Bereich der Verjüngung, d.h. zwischen dem Kolben und dem Gehäuse, ist in weiter vorteilhafter Weise eine Druckfeder angeordnet, gegen deren Kraft das Werkzeug in die Arbeitsposition schiebbar ist. Die Druckfeder wirkt somit zwischen dem Kolben und dem Gehäuse, so dass es zum Ausbringen des Werkzeugs erforderlich ist, die durch die Druckfeder definierte Vorspannung zu überwinden. Auch hier liegt eine Ähnlichkeit zu der Betätigung eines konventionellen Kugelschreibers vor.

Der zur Betätigung des Werkzeugs dienende Kolben ist im zylindrischen Bereich des Gehäuses geführt. Der verjüngte Bereich des Kolbens, der sich bis hin zur Gehäusespitze erstreckt, ist in weiter vorteilhafter Weise im Übergang zwischen dem zylindrischen Bereich des Gehäuses und der Gehäusespitze oder in der Gehäusespitze selbst geführt. Bei Betätigung lässt sich der verjüngte Bereich des Kolbens aus der Gehäusespitze heraus schieben, so dass sich das Werkzeug in der exponierten Arbeitsposition befindet.

In ganz besonders vorteilhafter Weise ist dem Kolben ein vorzugsweise orthogonal abragender Führungsstift zugeordnet, der in einer im Gehäuse ausgebildeten Führung läuft. Diese Führung ist als Ausklinkung bzw. Ausnehmung in der Gehäusewandung zu verstehen. Der Führungsstift kann an den Kolben angeschweißt oder in den Kolben eingeschraubt sein.

Die für den Führungsstift vorgesehene Führung ist im Konkreten als Schlitz in der Gehäusewandung ausgeführt. Zur Vermeidung einer unbeabsichtigten Betätigung, insbesondere zur Vermeidung eines unbeabsichtigten Herausschiebens des Werkzeugs in die Arbeitsposition, umfasst der Schlitz eine hintere Verrastung, in Form einer einen Anschlag bildenden Niesche. Des Weiteren kann eine vordere Verrastung gegen unbeabsichtigtes Hereinschieben bzw. Zurückfahren des Werkzeugs in die Aufbewahrungsposition vorgesehen sein, die entsprechend der Verrastung gegen unbeabsichtigtes Herausschieben mit umgekehrter Orientierung ausgeführt ist.

Der Führungsstift ist durch Drehen des Kolbens, vorzugsweise über das Betätigungsorgan, in einer entsprechenden axialen Position, aus der verrasteten Position heraus und in die verrastete Position hinein, drehbar. Dies bedeutet, dass eine axiale Betätigung zum Herausschieben und Zurückführen des Werkzeugs nicht ausreicht. Zur Sicherheit ist ein zusätzliches Drehen des Kolbens über das Betätigungsorgan erforderlich, wozu es sich anbietet, das Betätigungsorgan mit einer griffsicheren Riffelung oder dgl. zu versehen.

In Bezug auf die Ausgestaltung des Betätigungsorgans ist es von weiterem Vorteil, wenn dieses gegenüber dem Kolben vorzugsweise auf das Maß des Gehäuses verdickt bzw. erweitert ist. Ungeachtet einer die Handhabung begünstigenden Riffelung oder dgl. ist es denkbar, dass das Betätigungsorgan abgeflachte Bereiche aufweist, die einander gegenüberliegen. Über diese Bereiche lässt sich das Betätigungsorgan zum Drehen des Kolbens greifen, so dass eine Arretierung bzw. Entarretierung möglich ist.

Des Weiteren sei darauf hingewiesen, dass sich der Kolben gegen die Kraft der zuvor bereits erwähnten Feder bewegen lässt. Unter Zugrundelegung dieser Kraftbeaufschlagung ist es denkbar, dass die zur Führung dienenden Schlitze mit abgeschrägten endseitigen Flächen ausgestattet sind, so dass sich der Führungsstift quasi automatisch in die jeweils arretierende Position begibt, so dass zur abermaligen Betätigung stets eine Entarretierung bzw. ein Drehen des Kolbens über das Betätigungsorgan erforderlich ist. Mit dieser konstruktiven Maßnahme ist ein erhebliches Maß an Betriebssicherheit geschaffen.

Der Kolben, der verjüngte Bereich des Kolbens, das Betätigungsorgan und der Werkzeughalter können einteilig ausgeführt sein. Mehrteilige Ausführungen, beispielsweise auch unter Nutzung unterschiedlicher Materialien, sind ebenfalls denkbar.

Des Weiteren ist wesentlich, dass der Werkzeughalter eine Aufnahme für das Werkzeug umfasst, in die das Werkzeug - wie auch immer - eingebracht ist. Im einfachsten Falle ist das Werkzeug in die Aufnahme eingesteckt oder eingeklebt, insbesondere dann, wenn es sich bei dem Werkzeug um ein miniaturisiertes Diamantmesser handelt.

Das Gehäuse, der Kolben, das Betätigungsorgan und der Werkzeughalter können aus unterschiedlichen Materialien gefertigt sein, wobei es von Vorteil ist, die jeweiligen Bauteile aus Edelstahl und/oder aus Titan bzw. aus einer Titanlegierung zu fertigen. Eine hinreichende Biegefestigkeit ist im Rahmen solcher Werkstoffe gewährleistet, wobei sich diese Materialien insbesondere auch zur automatischen Reinigung in Waschmaschinen unter Nutzung einschlägiger Reinigungsmittel eignen, nämlich aufgrund der materialspezifischen Beständigkeit.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein Ausführungsbeispiel eines erfindungsgemäßen medizinischen Handgeräts, wobei es sich dabei um ein mit einem Diamantmesser ausgestattetes Schneidgerät für die Ophthalmologie handelt und wobei sich das Werkzeug in der eingeschobenen Aufbewahrungsposition befindet,
- Fig. 2: in einer schematischen Ansicht, geschnitten und gegenüber Fig. 1 um 90° um die Längsachse gedreht, den Gegenstand aus Fig. 1,
- Fig. 3: in einer schematischen Ansicht den Gegenstand aus den Fig. 1 und 2, in gleicher Positionierung wie in Fig. 1, wobei sich das Werkzeug in der exponierten bzw. herausgeschobenen Arbeitsposition befindet,
- Fig. 4: in einer schematischen Ansicht, geschnitten und gegenüber Fig. 3 um 90° um die Längsachse gedreht, den Gegenstand aus Fig. 3 und
- Fig. 5: in einer vergrößerten Ansicht einen Bereich des medizinischen Handgeräts aus den Fig. 1 bis 4, nämlich mit einem in der im Gehäuse ausgebildeten Führung befindlichen Führungsstift des Kolbens nebst Verrastung.

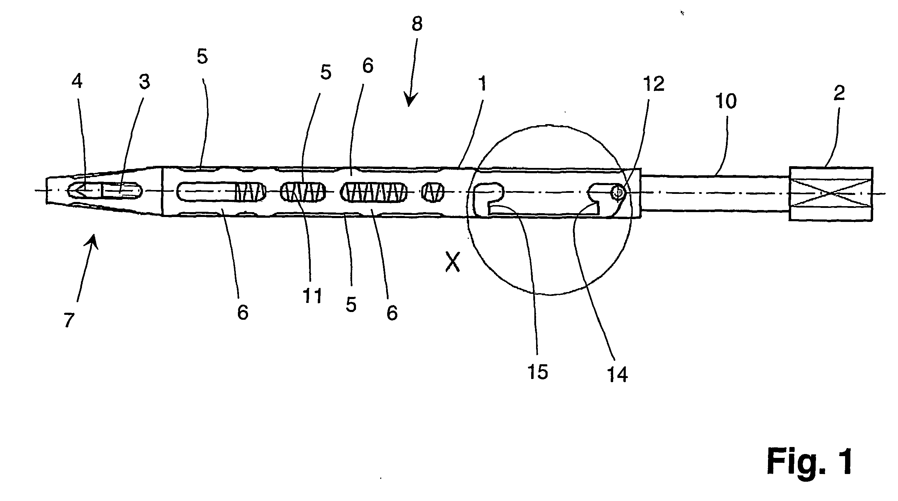

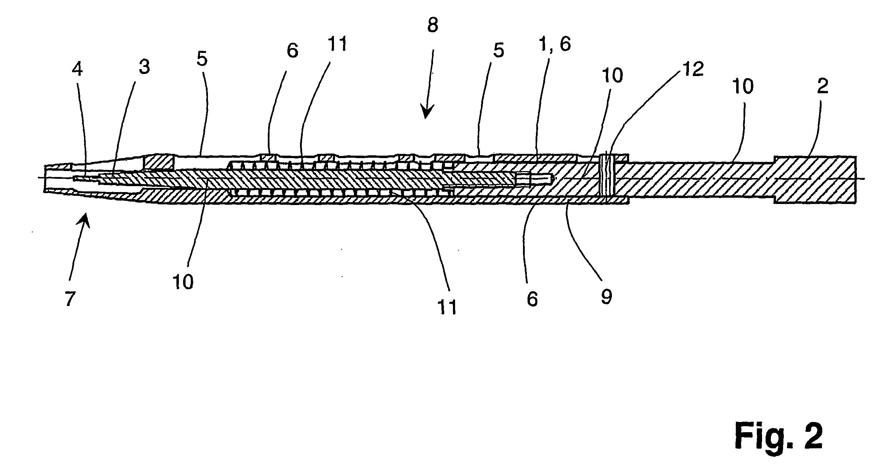

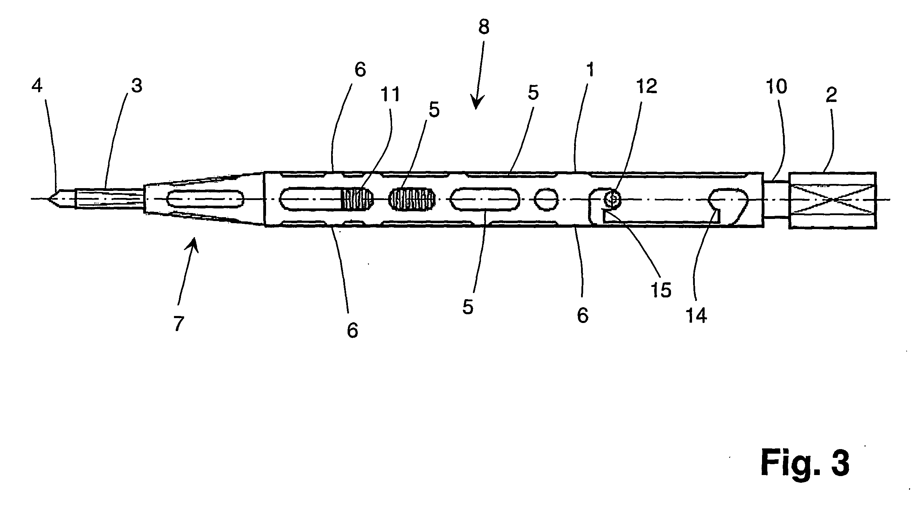

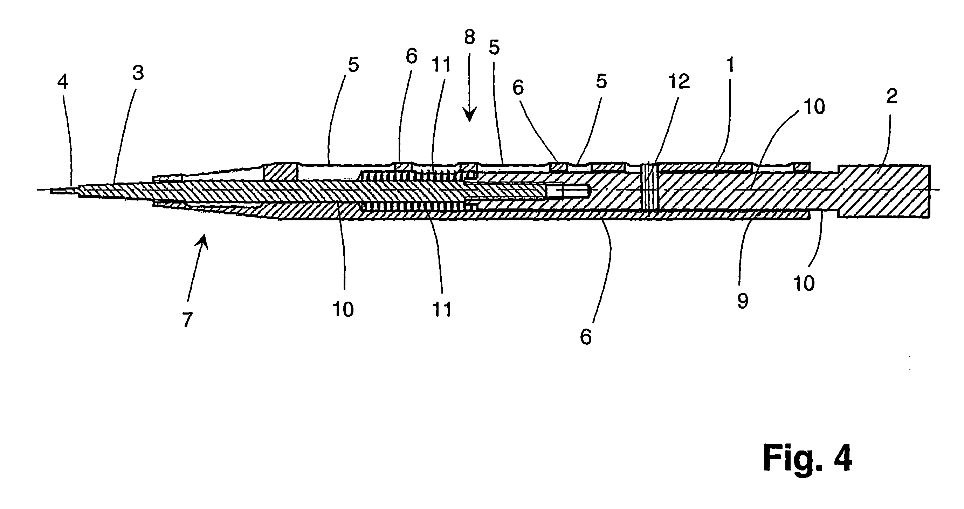

Fig. 1 zeigt in einer schematischen Ansicht ein Ausführungsbeispiel eines erfindungsgemäßen medizinischen Handgeräts, wobei es sich dabei im Konkreten um ein Schneidgerät für die Ophthalmologie handelt. Das Schneidgerät umfasst ein als Griffstück dienendes Gehäuse 1 und einen über ein Betätigungsorgan 2 in dem Gehäuse 1 verschiebbar angeordneten Werkzeughalter 3. Als Werkzeug 4 dient ein Diamant. Das Werkzeug 4 ist durch Betätigung des Betätigungsorgans 2 von einer in den Fig. 1 und 2 gezeigten Aufbewahrungsposition im Gehäuse 1 in eine in den Fig. 3 und 4 gezeigte Arbeitsposition außerhalb des Gehäuses 1 verschiebbar.

Das Gehäuse 1 weist im Bereich des in der Aufbewahrungsposition befindlichen Werkzeugs 4 Öffnungen 5 in der Gehäusewandung 6 auf.

Die Fig. 1 bis 4 zeigen gemeinsam, dass die Öffnungen 5 über die gesamte Länge des Gehäuses 1 hinweg ausgebildet sind. Auch lassen die Fig. erkennen, dass das Gehäuse 1 eine sich zum freien Ende hin stetig verjüngende Gehäusespitze 7 und einen zylindrischen Gehäuseschaft 8 aufweist. Die Öffnungen 5 sind sowohl in der Gehäusespitze 7 als auch im Gehäuseschaft 8 ausgebildet.

Die Öffnungen 5 sind über den gesamten Umfang des Gehäuses 1 hinweg ausgebildet, und zwar in axialer Richtung hintereinander fluchtend. Außerdem sind die Öffnungen 5 in ovaler Form mit abgerundeten Randbereichen ausgeführt. Die Öffnungen 5 sind derart dicht zueinander angelegt, dass sich die Gehäusewandung 6 im Sinne eines Gehäusekäfigs darstellt.

Die Fig. 2 und 4 lassen besonders deutlich erkennen, dass das Gehäuse 1 einen zylindrischen Durchgang 9 umfasst, der sich bis zur Gehäusespitze 7 hin erstreckt. Innerhalb des Durchgangs 9 ist ein den Werkzeughalter 3 tragender Kolben 10 verschiebbar. Außerdem ist wesentlich, dass sich der Kolben 10 zum Werkzeughalter 3 hin verjüngt, wobei im Bereich der Verjüngung, d.h. im vorderen Bereich, nämlich zwischen dem Kolben 10 und dem Gehäuse 1, eine Druckfeder 11 angeordnet ist, gegen deren Kraft das Werkzeug 4 in die Arbeitsposition verbringbar ist.

Der Kolben 10 ist im zylindrischen Bereich des Gehäuses 1, d.h. im Gehäuseschaft 8, und der verjüngte Bereich des Kolbens 10 ist im Übergang zwischen dem zylindrischen Bereich des Gehäuses 1 und der Gehäusespitze 7 geführt, so dass sich insgesamt eine optimale, d.h. kippsichere Führung des Kolbens 10 ergibt.

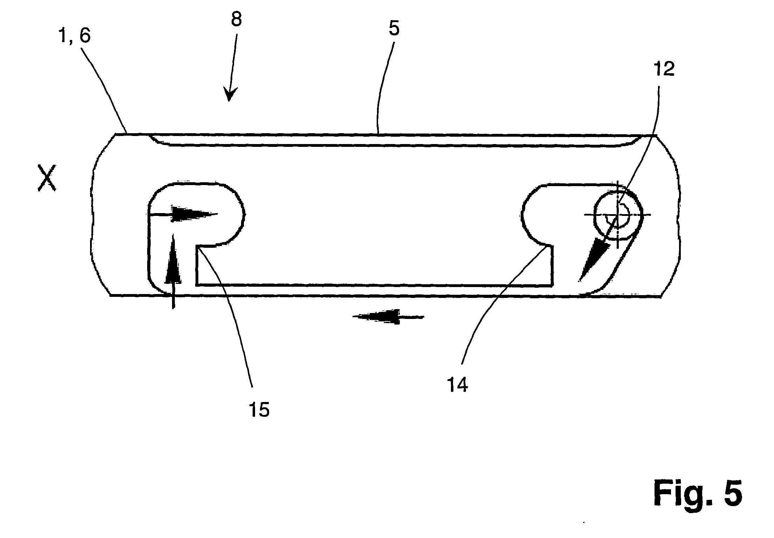

Die Fig. 1, 3 und 5 zeigen deutlich, dass dem Kolben 10 ein orthogonal abragender Führungsstift 12 zugeordnet ist, der in einer im Gehäuse 1 ausgebildeten Führung 13 läuft. Der Führungsstift 12 ist in den Kolben 1 eingeschraubt.
Die Führung 13 ist als Schlitz in der Gehäusewandung 6 ausgeführt.

Die Fig. 1, 3 und 5 zeigen des Weiteren deutlich, dass die Führung 13 bzw. der Schlitz eine hintere Verrastung 14 gegen unbeabsichtigtes Herausschieben des Werkzeugs 4 in die Arbeitsposition aufweist. Des Weiteren ist eine vordere Verrastung 15 gegen unbeabsichtigtes Hereinschieben des Werkzeugs 4 in die Aufbewahrungsposition vorgesehen. Beide Verrastungen 14, 15 sind derart ausgelegt und ausgebildet, dass der Führungsstift 12 durch Drehen des Kolbens 10, nämlich über das Betätigungsorgan 2, in einer entsprechenden axialen Position, aus der verrasteten Position, nämlich aus der hinteren Verrastung 14 heraus, und nach dem Herausschieben des Werkzeugs durch axiale Betätigung des Betätigungsorgans 2 in eine verrastete Position, nämlich in die vordere Verrastung 15 hinein, drehbar und dort jeweils arretierbar bzw. verankerbar ist. Das jeweilige Lösen aus den Verrastungen 14, 15 erfolgt entsprechend umgekehrt.

Hinsichtlich weiterer Merkmale, die sich den Figuren nicht entnehmen lassen, sei zur Vermeidung von Wiederholungen auf den allgemeinen Teil der Beschreibung verwiesen.

Schließlich sei angemerkt, dass das voranstehend erörterte Ausführungsbeispiel lediglich der beispielhaften Erläuterung des erfindungsgemäßen Handgeräts dient, dieses jedoch nicht auf das Ausführungsbeispiel einschränkt.

## Patentansprüche

1. Medizinisches Handgerät, insbesondere chirurgisches Schneidgerät zur Anwendung in der Ophthalmologie, mit einem als Griffstück dienenden Gehäuse (1) und einem über ein Betätigungsorgan (2) in dem Gehäuse (1) verschiebbar angeordneten Werkzeughalter (3) mit einem Werkzeug (4), insbesondere für ein Messer, wobei das Werkzeug (4) durch Betätigung des Betätigungsorgans (2) von einer Aufbewahrungsposition im Gehäuse (1) in eine Arbeitsposition außerhalb des Gehäuses (1) verschiebbar ist, wobei das Gehäuse (1) zumindest im Bereich des in der Aufbewahrungsposition befindlichen Werkzeugs (4) Öffnungen (5) in der Gehäusewandung (6) aufweist und wobei das Gehäuse (1) über die gesamte Länge hinweg Öffnungen (5) in der Gehäusewandung (6) aufweist,
**dadurch gekennzeichnet, dass** die Öffnungen (5) allseitig, d.h. über den gesamten Umfang hinweg, im Gehäuse (1) ausgebildet sind.

2. Handgerät nach Anspruch 1, **dadurch gekennzeichnet, dass** das Gehäuse (1) eine sich zum freien Ende hin vorzugsweise stetig verjüngende Gehäusespitze (7) und einen vorzugsweise zylindrischen Gehäuseschaft (8) aufweist und dass die Öffnungen (5) sowohl in der Gehäusespitze (7) als auch im Gehäuseschaft (8) ausgebildet sind.

3. Handgerät nach Anspruch 1 oder 2, **dadurch gekennzeichnet, dass** die Öffnungen (5) in axialer Richtung versetzt zueinander oder
in axialer Richtung hintereinander fluchtend ausgebildet sind.

4. Handgerät nach einem der Ansprüche 1 bis 3, **dadurch gekennzeichnet, dass** die Öffnungen (5) im Wesentlichen rund oder
schlitzartig, vorzugsweise in ovaler Form, ausgebildet sind und/oder dass die Öffnungen (5) derart ausgebildet sind, dass das Gehäuse (1) ein Gehäusekäfig bildet.

5. Handgerät nach einem der Ansprüche 1 bis 4, **dadurch gekennzeichnet, dass** das Gehäuse (1) bis zur Gehäusespitze (7) einen zylindrischen Durchgang (9) aufweist, in dem ein den Werkzeughalter (3) tragender Kolben (10) verschiebbar angeordnet ist.

6. Handgerät nach Anspruch 5, **dadurch gekennzeichnet, dass** sich der Kolben (10) zum Werkzeughalter (3) hin, vorzugsweise über eine Abstufung, verjüngt und dass im Bereich der Verjüngung, d.h. zwischen dem Kolben (10) und dem Gehäuse (1), eine Druckfeder (11) angeordnet ist, gegen deren Kraft das Werkzeug (4) in die Arbeitsposition verbringbar ist.

7. Handgerät nach Anspruch β, **dadurch gekennzeichnet, dass** der Kolben (10) im zylindrischen Bereich des Gehäuses (1) und der verjüngte Bereich des Kolbens (10) im Übergang zwischen dem zylindrischen Bereich des Gehäuses (1) und der Gehäusespitze (7) oder in der Gehäusespitze (7) geführt ist.

8. Handgerät nach Anspruch 6 oder 7, **dadurch gekennzeichnet, dass** dem Kolben (10) ein vorzugsweise orthogonal abragender Führungsstift (12) zugeordnet ist, der in einer im Gehäuse (1) ausgebildeten Führung (13) läuft.

9. Handgerät nach Anspruch 8, **dadurch gekennzeichnet, dass** der Führungsstift (12) in den Kolben (10) eingeschraubt ist und/oder dass
die Führung (13) als Schlitz in der Gehäusewandung (6) ausgeführt ist.

10. Handgerät nach Anspruch 9, **dadurch gekennzeichnet, dass** der Schlitz (13) eine hintere Verrastung (14) gegen unbeabsichtigtes Herausschieben des Werkzeugs (4) in die Arbeitsposition und eine vordere Verrastung (15) gegen unbeabsichtigtes Hereinschieben des Werkzeugs (4) in die Aufbewahrungsposition umfasst, wobei
der Führungsstift (12) durch Drehen des Kolbens (10), vorzugsweise über das Betätigungsorgan (2), in einer entsprechenden axialen Position, aus der verrasteten Position heraus und in die verrastete Position hinein drehbar sein kann.

11. Handgerät nach einem der Ansprüche 5 bis 10, **dadurch gekennzeichnet, dass** das Betätigungsorgan (2) gegenüber dem Kolben (10) vorzugsweise auf das Maß des Gehäuses (1) verdickt bzw. erweitert ist und gegenüberliegende, abgeflachte Flächen aufweist.

12. Handgerät nach einem der Ansprüche 5 bis 11, **dadurch gekennzeichnet, dass** der Kolben (10), der verjüngte Bereich des Kolbens (10), das Betätigungsorgan (2) und der Werkzeughalter (3) einteilig ausgeführt sind.

13. Handgerät nach einem der Ansprüche 1 bis 12, **dadurch gekennzeichnet, dass** der Werkzeughalter (3) eine Aufnahme für das Werkzeug (4) umfasst, in die das Werkzeug (4) eingeklebt ist, wobei
das Werkzeug (4) als Diamantmesser ausgeführt sein kann.

14. Handgerät nach einem der Ansprüche 1 bis 13, **dadurch gekennzeichnet, dass** das Gehäuse (1), der Kolben (10), das Betätigungsorgan (2) und der Werkzeughalter (3) aus Edelstahl oder aus
Titan bzw. aus einer Titanlegierung gefertigt sind.

## Claims

1. Hand-held medical device, in particular a surgical cutting device for use in ophthalmology, having a housing (1) which acts as a gripping member, and a tool holder (3) which is arranged so as to be able to be displaced in the housing (1) by means of an actuation member (2) and which has a tool (4), in particular for a blade, the tool (4) being able to be displaced by the actuation member (2) being actuated from a storage position in the housing (1) into an operating position outside the housing (1), the housing (1) having openings (5) in the housing wall (6) at least in the region of the tool (4) located in the storage position, and the housing (1) having openings (5) in the housing wall (6) over the entire length,
**characterised in that** the openings (5) are formed in the housing (1) at all sides, that is to say, over the entire periphery.

2. Hand-held device according to claim 1, **characterised in that** the housing (1) has a housing tip (7) which preferably tapers continuously in the direction towards the free end and a preferably cylindrical housing shaft (8) and **in that** the openings (5) are formed both in the housing tip (7) and in the housing shaft (8).

3. Hand-held device according to claim 1 or claim 2, **characterised in that** the openings (5) are constructed so as to be offset with respect to each other in an axial direction or so as to be in alignment one behind the other in an axial direction.

4. Hand-held device according to any one of claims 1 to 3, **characterised in that** the openings (5) are constructed so as to be substantially round or slot-like, preferably oval, and/or **in that** the openings (5) are constructed in such a manner that the housing (1) forms a housing cage.

5. Hand-held device according to any one of claims 1 to 4, **characterised in that** the housing (1) has, as far as the housing tip (7), a cylindrical aperture (9) in which a piston (10) which carries the tool holder (3) is displaceably arranged.

6. Hand-held device according to claim 5, **characterised in that** the piston (10) tapers in the direction towards the tool holder (3), preferably via a stepped portion, and **in that** there is arranged in the region of the tapering, that is to say, between the piston (10) and the housing (1), a compression spring (11) counter to the force of which the tool (4) can be moved into the operating position.

7. Hand-held device according to claim 6, **characterised in that** the piston (10) is guided in the cylindrical region of the housing (1) and the tapered region of the piston (10) is guided in the transition between the cylindrical region of the housing (1) and the housing tip (7) or in the housing tip (7) .

8. Hand-held device according to claim 6 or claim 7, **characterised in that** there is associated with the piston (10) a guiding pin (12) which preferably protrudes in an orthogonal manner and which travels in a guide (13) which is formed in the housing (1).

9. Hand-held device according to claim 8, **characterised in that** the guiding pin (12) is screwed into the piston (10) and/or **in that** the guide (13) is constructed as a slot in the housing wall (6).

10. Hand-held device according to claim 9, **characterised in that** the slot (13) comprises a rear catch (14) to prevent the tool (4) from being unintentionally pushed out into the operating position and a front catch (15) to prevent the tool (4) from being unintentionally pushed into the storage position,
the guiding pin (12), by rotating the piston (10), preferably via the actuation member (2), in a corresponding axial position, being able to be rotatable out of the locked position and into the locked position.

11. Hand-held device according to any one of claims 5 to 10, **characterised in that** the actuation member (2) is thickened or expanded with respect to the piston (10), preferably to the dimension of the housing (1), and has opposing flattened faces.

12. Hand-held device according to any one of claims 5 to 11, **characterised in that** the piston (10), the tapered region of the piston (10), the actuation member (2) and the tool holder (3) are constructed in one piece.

13. Hand-held device according to any one of claims 1 to 12, **characterised in that** the tool holder (3) comprises a receiving member for the tool (4), in which the tool (4) is adhesively-bonded,
the tool (4) being able to be constructed as a diamond blade.

14. Hand-held device according to any one of claims 1 to 13, **characterised in that** the housing (1), the piston (10), the actuation member (2) and the tool holder (3) are produced from high-grade steel or from titanium or a titanium alloy.

## Revendications

1. Instrument médical à main, en particulier instrument chirurgical de coupe pour une utilisation en ophtalmologie, comprenant un boîtier (1) servant de poignée et un porte-outil (3) disposé coulissant via un organe d'actionnement (2) dans le boîtier (1), avec un outil (4), en particulier un couteau, l'outil (4) pouvant être coulissé par actionnement de l'organe d'actionnement (2) d'une position de rangement dans le boîtier (1) dans une position de travail en dehors du boîtier (1), le boîtier (1) comportant des ouvertures (5) dans la paroi du boîtier (6), au moins dans la zone de l'outil (4) se trouvant dans la position de rangement, et le boîtier (1) comportant sur toute la longueur des ouvertures (5) dans la paroi du boîtier (6),
**caractérisé en ce que** les ouvertures (5) sont configurées dans le boîtier (1) dans toutes les directions, c'est-à-dire sur toute la périphérie.

2. Instrument à main selon la revendication 1, **caractérisé en ce que** le boîtier (1) comporte une pointe de boîtier (7) s'effilant de préférence constamment vers l'extrémité libre, et une tige de boîtier (8), de préférence cylindrique, et **en ce que** les ouvertures (5) sont pratiquées aussi bien dans la pointe de boîtier (7) que dans la tige de boîtier (8).

3. Instrument à main selon la revendication 1 ou 2, **caractérisé en ce que** les ouvertures (5) sont configurées décalées les unes des autres dans la direction axiale
ou sur une même ligne dans la direction axiale.

4. Instrument à main selon l'une quelconque des revendications 1 à 3, **caractérisé en ce que** les ouvertures (5) sont configurées sensiblement rondes
ou en fente, de préférence de forme ovale et/ou **en ce que** les ouvertures (5) sont configurées de telle sorte que le boîtier (1) forme une cage de boîtier.

5. Instrument à main selon l'une quelconque des revendications 1 à 4, **caractérisé en ce que** le boîtier (1) comporte jusqu'à la pointe du boîtier (7) un passage cylindrique (9), dans lequel un piston (10) portant le porte-outil (3) est disposé coulissant.

6. Instrument à main selon la revendication 5, **caractérisé en ce que** le piston (10) se rétrécit en direction du porte-outil (3), de préférence par un décrochement, et qu'un ressort de compression (11) est disposé dans la zone du rétrécissement, c'est-à-dire entre le piston (10) et le boîtier (1), contre la force duquel l'outil (4) peut être amené dans la position de travail.

7. Instrument à main selon la revendication 6, **caractérisé en ce que** le piston (10) est guidé dans la zone cylindrique du boîtier (1) et **en ce que** la zone rétrécie du piston (10) est guidée dans la transition entre la zone cylindrique du boîtier (1) et la pointe du boîtier (7), ou dans la pointe du boîtier (7).

8. Instrument à main selon la revendication 6 ou 7, **caractérisé en ce qu'**une tige de guidage (12) dépassant de préférence orthogonalement, qui glisse dans un guide (13) pratiqué dans le boîtier (1), est affectée au piston (10).

9. Instrument à main selon la revendication 8, **caractérisé en ce que** la tige de guidage (12) est vissée dans le piston (10) et/ou **en ce que**
le guide (13) est exécuté comme fente dans la paroi du boîtier (6).

10. Instrument à main selon la revendication 9, **caractérisé en ce que** la fente (13) comprend un encliquetage arrière (14) pour empêcher une sortie intempestive de l'outil (4) par coulissement dans la position de travail et un encliquetage avant (15) pour empêcher une rentrée intempestive de l'outil (4) par coulissement dans la position de rangement, sachant que
la tige de guidage (12), dans une position axiale adéquate, peut pouvoir être tournée par rotation du piston (10), de préférence par l'intermédiaire de l'organe d'actionnement (2), pour sortir de la position encliquetée et pour rentrer dans la position encliquetée.

11. Instrument à main selon l'une quelconque des revendications 5 à 10, **caractérisé en ce que** l'organe d'actionnement (2) est épaissi, respectivement élargi, par rapport au piston (10), de préférence à la cote du boîtier (1), et comporte des surfaces aplanies en vis-à-vis.

12. Instrument à main selon l'une quelconque des revendications 5 à 11, **caractérisé en ce que** le piston (10), la partie rétrécie du piston (10), l'organe d'actionnement (2) et le porte-outil (3) sont réalisés en une seule pièce.

13. Instrument à main selon l'une quelconque des revendications 1 à 12, **caractérisé en ce que** le porte-outil (3) comprend un logement pour l'outil (4), dans lequel l'outil (4) est collé, sachant que l'outil (4) peut être exécuté comme couteau à diamant.

14. Instrument à main selon l'une quelconque des revendications 1 à 13, **caractérisé en ce que** le boîtier (1), le piston (10), l'organe d'actionnement (2) et le porte-outil (3) sont fabriqués en acier inoxydable ou
en titane, respectivement en un alliage de titane.
